# EUROPEAN PATENT SPECIFICATION

(11) **EP 1 193 998 B1**
(45) Date of publication and mention of the grant of the patent: **05.08.2009**
(21) Application number: 01123671.8
(22) Date of filing: 02.10.2001
(51) Int. Cl.: H04R 1/00, H04R 25/00

(54) **Living organism conductive actuator**
Leitender Aktuator für lebenden Organismus
Actionneur conducteur pour organisme vivant

(30) Priority: 02.10.2000 JP 2000302736
(43) Date of publication of application: 03.04.2002
(73) Proprietor: NTT DoCoMo, Inc., Tokyo 100-6150 (JP)
(72) Inventor: Uchida, Koji, Taihaku-ku Sendai-shi Miyagi 982-8510 (JP); Fukumoto, Masaaki, Yokohama-shi, Kanagawa-ken, 235-0033 (JP); Sugimura, Toshiaki, Yokohama-shi, Kanagawa-ken, 236-0057 (JP)
(74) Representative: Hofer, Dorothea

(56) References cited:
- EP-A- 0 866 592
- EP-A- 0 951 883
- WO-A-00/13329
- PATENT ABSTRACTS OF JAPAN vol. 1999, no. 13, 30 November 1999 (1999-11-30) -& JP 11 215213 A (RICOH MICRO ELECTRONICS KK), 6 August 1999 (1999-08-06)
- PATENT ABSTRACTS OF JAPAN vol. 2000, no. 09, 13 October 2000 (2000-10-13) -& JP 2000 166962 A (IFUKUBE TATSU), 20 June 2000 (2000-06-20)
- PATENT ABSTRACTS OF JAPAN vol. 2000, no. 03, 30 March 2000 (2000-03-30) -& JP 11 355870 A (ONKYOO RIBU KK), 24 December 1999 (1999-12-24)

## Description

### Background of the Invention:

### (1) Field of the Invention

The present invention relates to a living organism conductive actuator constructed by a portion for transmitting a communication signal, such as an audio signal, to a living organism as a vibrating medium and a voice input portion, and more particularly, relates to the living organism conductive actuator used in an input and an output for transmitting and receiving a calling signal of a portable telephone, etc., and the communication signal, such as an audio signal.

### (2) Background Art

A living organism conductive actuator is conventionally known as a device used to receive a calling signal of a portable telephone, etc., and transmit and receive a communication signal. The living organism conductive actuator is integrally constructed by a structure in which a communication signal transmitting portion for transmitting the received communication signal, such as an audio signal, and a voice input portion are overlapped in an axial direction. The communication signal transmitting portion transmits the received communication signal, such as an audio signal, to the interior of an external cover through which two wires for support extend.

In the conventional living organism conductive actuator, the structure for overlapping the communication signal transmitting portion and the voice input portion having an external surface formed by rubber in the axial direction, i.e., a longitudinal structure has an influence of a reduction in sound leakage, vibrational transmission, noises of a voice input and an external mechanical load on internal constructional parts.

EP 0 951 883 A1 describes a wearable communication device including a bone conduction actuator which is applicable for being in contact with a user's wrist, hand, back of the hand, finger or nail in order to transmit voice signals. The user inserts the user's finger into the user's ear canal, or touches the user's finger to a part near the user's ear, or puts the user's fingertip or nail on the user's ear canal so as to block the user's ear canal when the user uses the wearable communication device. Also, the wearable command input device includes a first part for detecting shock or acceleration which arises when the user taps the user's fingertip on the surface of an object or when the user taps the fingertips mutually and which is transmitted through the user's finger, a second part for detecting specific frequency components which are included in signals from the first part and for detecting the presence or the absence of the tap of the finger of the user, and a part for determining and executing commands based on series of signals output from the second part.

WO 00/13329 A1 describes a personal communications apparatus comprising a first part, having a microphone, and a second part, having a loudspeaker. The first part is adapted to be worn on a user's wrist and the second part is adapted to be worn on their finger. In use, the user's hand is held up to the side of their head, thereby placing the microphone adjacent to their mouth and the loudspeaker adjacent to their ear. Communication between first and second parts of the apparatus is by means of signals transmitted via the user's skin. In a non-illustrated embodiment the second part is adapted to be worn on the ear.

JP 11-215213 A describes a device to surely report an incoming state to a user without bothering the nearby persons by receiving the radio waves sent to a base station from a portable telephone set which is kept in an incoming state and by reporting this incoming state to the user by applying a prescribed signal to the user's body via the electric stimuli which are caused by contact between the user and an incoming reporting part. A main body cover includes a display part which shows the time in numeric characters, a receiving part which receives the radio waves sent to a base station from a portable telephone set that is kept in an incoming state, and an incoming report control part which controls the voltage applied to a pair of electrodes by the signal received from the receiving part. In regard to a wristwatch, the electric stimulus adding voltage is applied to the electrodes when the radio waves sent to the base station from the portable telephone set kept in an incoming state are received. Then the gradient of applied voltage is added to a part of a human body such as a wrist, etc., touching the electrodes as the safe electric stimuli. Thus, the incoming state of the portable telephone set is reported via the electric stimuli.

EP 0 866 592 A2 describes an array of stimulators associated with a personal communications device for providing the user with tactile messaging respecting call processing or call network status. The array is positioned on the device so as to be in contact with the user while the terminal is carried or worn, on a wrist, for example. The stimulators of the array, are activated independently so as to provide the user with an encoded message of call processed such as alerting, dial tone, busy signal, etc. Preferably each status is associated with one of a set of unique patterns of operation of the stimulators recognizable by the user as tactile image or pattern of operation, rather than necessitating tactile sensation of individual sequences of each stimulator. Advantageously audio and haptic signaling is synchronized to provide a tactile warning to alert user to impending audio signal, and thereby allow a lower level, i.e. quieter, audio signal to be used, because the user is primed by the tactile signal to expect the audio signaling. Thus the audio threshold for effective signaling is reduced and obtrusiveness of audio signaling in public places may be reduced when used in combination with tactile messaging.

JP 2000-166962 A describes a device and method to precisely transmit a speech signal and to make convenient for portable use by converting an input speech signal to a precise vibration pattern to vibrate each vibration pin of a vibrator piece matrix independent from each other to continuously supply a vibration pattern to a finger tip. A side suppressing and combining part obtained by connecting a band filter and a critical band filter to a microphone reinforces spatial resolution of a low tactile sense and executes signal emphasizing processing simulating side suppressing processing executed by a vital nervous system. Thus, the formant of a vowel and a consonant part are intensified. Next, in order to make stimulation flow from right to left on a finger tip, a 16/64 sweep conversion part gives time delay to data driving the vibration pins of horizontally four lines to synchronize-output by an analog quantity. This data is inputted to a tactile sense part through amplifiers, 4x 16ch to vertically move each vibration pin to transmit to the finger tip. Thus, the speech signal is precisely transmitted by a small device.

### Summary of the Invention:

It is an object of the present invention to provide a living organism conductive actuator having an optimal structure able to reduce noise propagation (hereinafter called sound leakage) due to a medium on an external surface and improve transmission characteristics and control a vibration transmitting path of internal and external portions.

The object is attained by a living organism conductive actuator according to claim 1. Further developments of the invention are specified in the dependent claims, respectively.

Further features and advantages of the invention will arise from the description of embodiments with reference to the enclosed figures.

### Brief Description of the Drawings

Fig. 1 A is a front view showing a living organism conductive actuator of a longitudinal structure in the prior art;
Fig. 1 B is a plan semi-sectional view of an actuator of Fig. 1 A;
Fig. 1C is a cross-sectional view taken along line 1C-1C of Fig. 1 A;
Fig. 1D is a cross-sectional view taken along line 1D-1D of Fig. 1 A;
Fig. 2A is a plan view showing a living organism conductive actuator in an embodiment of the present invention;
Fig. 2B is a front view of the actuator of Fig. 2A;
Fig. 2C is a bottom view of the actuator of Fig. 2A;
Fig. 2D is a cross-sectional view taken along line IID-IID of the actuator of Fig. 2A;
Fig. 3A is a graph showing a reduction in input noise due to the arrangement of a voice input portion of a conventional structure;
Fig. 3B is a graph showing a reduction in input noise due to the arrangement of a voice input portion of a structure of the invention;
Fig. 4 is a graph showing an analyzed presumed value of sound leakage spl due to a change in weight of an external cover of the living organism conductive actuator in an embodiment of the invention;
Fig. 5 is a graph showing experimental results of the sound leakage spl due to the change in weight of the external cover of the living organism conductive actuator in the embodiment of the invention;
Fig. 6 is a graph showing experimental results of the sound leakage spl of a two-resonance structure;
Fig. 7 is a graph showing experimental results of high frequency sound leakage spl due to a change in a joining member; and
Figs. 8A to 8D are views respectively showing various kinds of modified examples of the structure of the living organism conductive actuator in the embodiment of the invention.

### Description of the Preferred Embodiment:

A living organism conductive actuator in the prior art will be explained with reference to the drawings to easily understand the invention prior to the description of an embodiment of the invention.

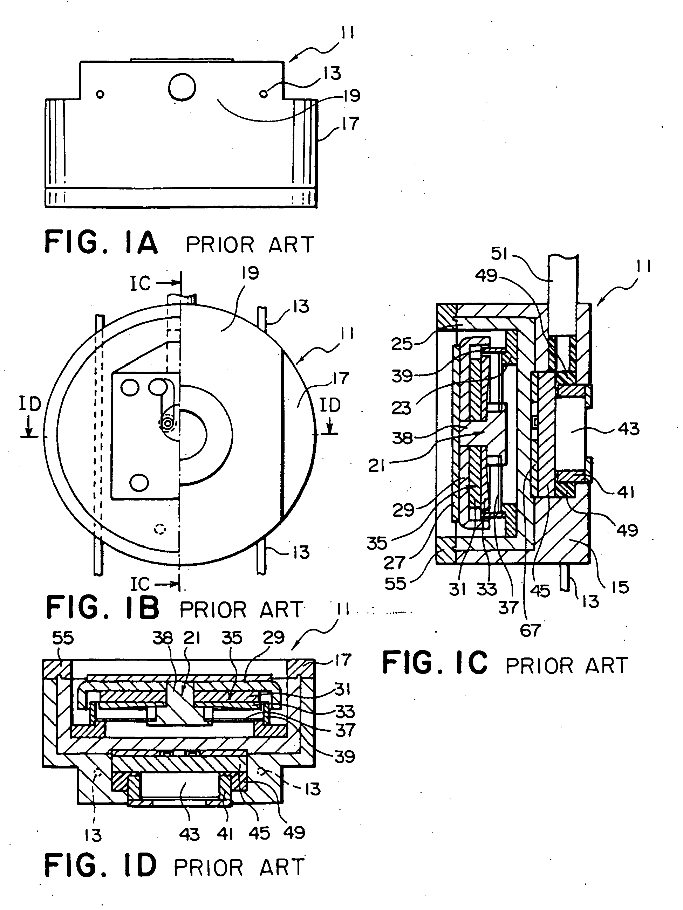

Referring to Figs. 1A to 1D, a living organism conductive actuator 11 is integrally constructed by a structure in which a communication signal transmitting portion 17 and a voice input portion 19 are overlapped with each other in an axial direction. The communication signal transmitting portion 17 transmits a received communication signal, such as an audio signal, to the interior of an external cover 15 through which two wires 13 for support extend. In the communication signal transmitting portion 17, a living organism contact signal transmitting portion 21 opened to one end portion of the external cover 15 and a base 23 for supporting the living organism contact signal transmitting portion 21 are arranged within a living organism contact vibration transmitting portion cover 25. In the living organism contact signal transmitting portion 21, a magnetic circuit 35 constructed by overlapping a yoke 29 of a cup shape, a permanent magnet 31 and a fixed member 33 is overlapped with a plate 27 of a resin shape. Further, a spiral spring 37 is overlapped with the magnetic circuit 35 and a fixed member 38 extends through the magnetic circuit 35 so that the plate 27, the magnetic circuit 35 and the fixed member 38 are integrally formed. The spiral spring 37 is fixed to the living organism contact vibration transmitting portion cover 25 through the base 23. A coil 39 is mounted between the magnetic circuit 35 and the base 23.

The voice input portion 19 has a vibrationproof material 41 arranged so as to be exposed on the other end face side of the external cover 15, a microphone 43 inserted into the vibrationproof material 41, and a substrate 45 stuck to the other face of the microphone 43 through an elastic adhesive 49. An internal circuit 67 is arranged on a face of the substrate 45 on a side opposed to the microphone 43. The internal circuit 67 is mounted to an outside face of the living organism contact vibration transmitting portion cover 25.

The embodiment of the invention will next be explained with reference to the drawings. In the explanation of the present invention, similar parts are described by using similar reference numerals.

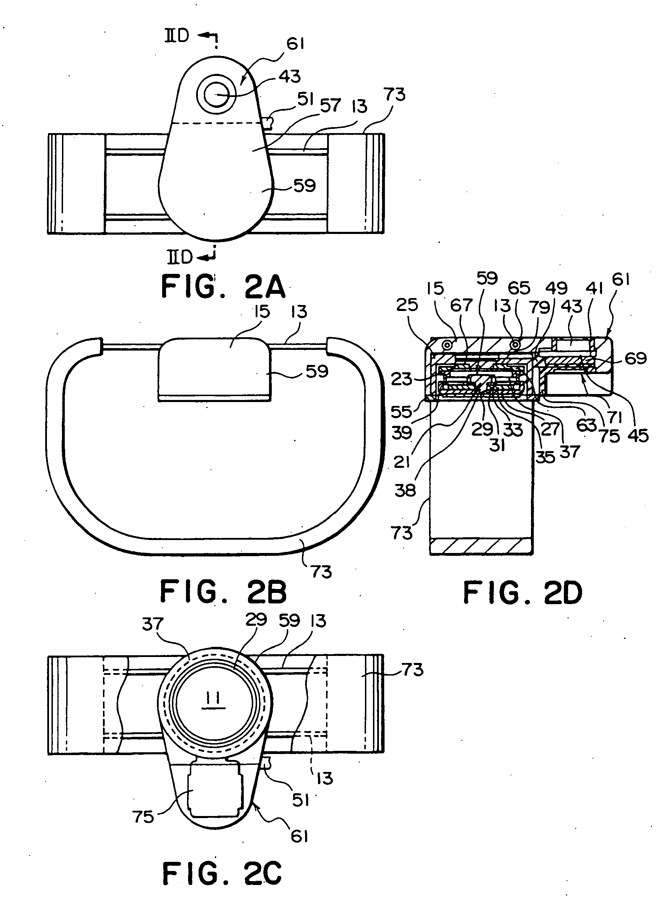

Referring to Figs. 2A to 2D, a living organism conductive actuator 57 is constructed by a communication signal transmitting portion 59 for transmitting a received voice signal and a received calling signal by contact, and a voice input portion 61.

The communication signal transmitting portion 59 has a living organism contact vibration transmitting portion 21 and an internal circuit 67 arranged on the outside face of a living organism vibration contact portion cover 25 covering the living organism contact vibration transmitting portion 21. The living organism contact vibration transmitting portion 21 and the internal circuit 67 are covered with the external cover 15.

The living organism contact vibration transmitting portion 21 has the magnetic circuit 35 arranged on one face of the plate 27 of a resin shape, the spiral spring 37 for supporting the magnetic circuit 35, and the base 23 for supporting the spiral spring 37. The coil 39 is inserted between the base 23 arranged around the spiral spring 37 and the magnetic circuit 35. The base 23 is supported on an inside face of the living organism contact vibration transmitting portion cover 25 through a spring 63 of a resin shape and a material 79 having a large Poisson's ratio. Thus, the plate 27 of a resin shape is used in a living organism contact portion of the communication signal transmitting portion 59 so that a feeling of physical disorder can be prevented at a using time of an operator and friction in a shearing direction can be reduced.

The magnetic circuit 35 has the yoke 29 of the cap shape, the permanent magnet 31 of a disk shape, and the supporting portion 33 for supporting the permanent magnet 31, and is integrally fixed in this order by the fixed portion 38 together with the spiral spring 37.

The voice input portion 61 is adjacent to the communication signal input portion 59 within the external cover 25, and has the microphone 43 stored into the vibrationproof material 41, the substrate 45 for supporting the microphone 43 on one face thereof, and the supporting member 69 for supporting the substrate 45. The members are stored into the external cover 15 through an elastic adhesive 49. A gap 71 is formed on a lower side of the external cover 15. The gap 71 is formed to avoid a mechanical interference of a change in living organism shape due to bending when the living organism conductive actuator is used by an operator's wrist, etc.

A main body of the living organism conductive actuator 57 is supported by two wires 13,13. An outer circumferential portion of the wire 13 is guided by the viscoelastomer 65 so that the generation of an additional resonance is structurally restrained.

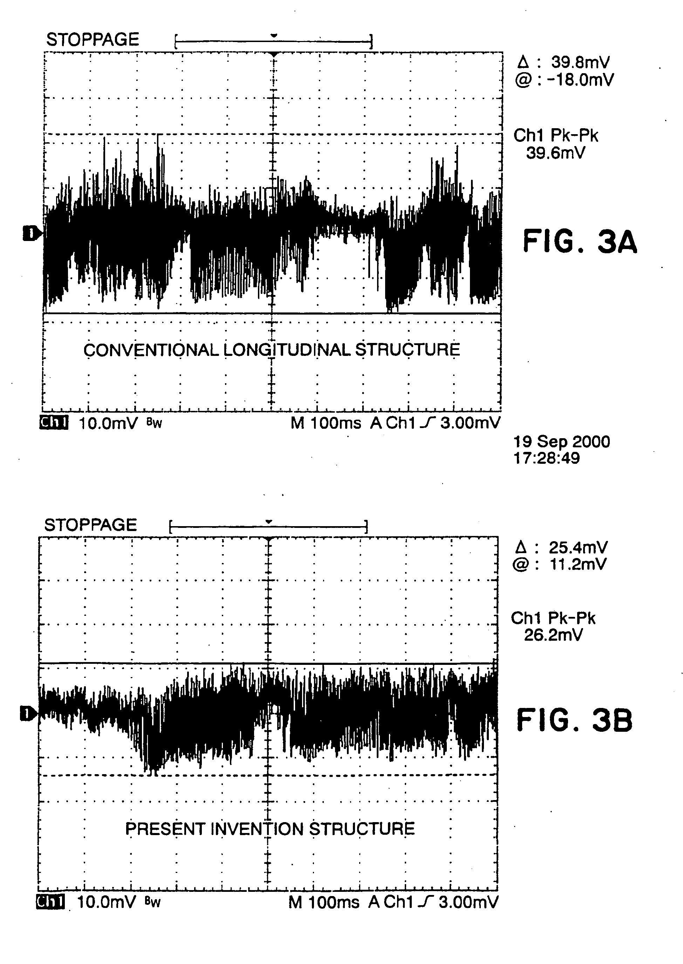

As can be seen by comparing Figs. 3A and 3B, a sound input noise can be reduced by moving an arrangement of the voice input portion 61 from an upper portion of the communication signal transmitting portion 59 to its side face portion in the structure of the invention. Further, the voice input noise transmitted via the external cover 15 is reduced by adding the microphone 43 in the voice input portion 61 and the vibrationproof material 41 around the substrate 45.

Vibration insulation and close contact with an living organism are improved by using the material 55 having a vibrationproof effect in a living organism contact portion except for the communication signal transmitting portion 59 such that no vibration transmitted to the living organism, e.g., an operator's wrist, hand, hand rear portion, finger, or nail tip, etc. is returned to the external cover 15.

It is possible to restrain noises due to a mechanical load, such as manual, crushing with respect to the internal circuit 67 of the communication signal transmitting portion 59 by manufacturing the external cover 15 by a metal. In addition, it is possible to restrain damage due to the mechanical load, such as manual, crushing with respect to the internal circuit 67 by manufacturing the external cover 15 by a metal.

The weight of a main body of the living organism conductive actuator 57 is set to several ten grams and is about 2.5 times the conventional weight to reduce noise propagation (reduce sound leakage) due to a medium on an external surface so that loss due to the weight in kinetic energy is increased and a vibrational displacement on a side of the external cover 15 is reduced.

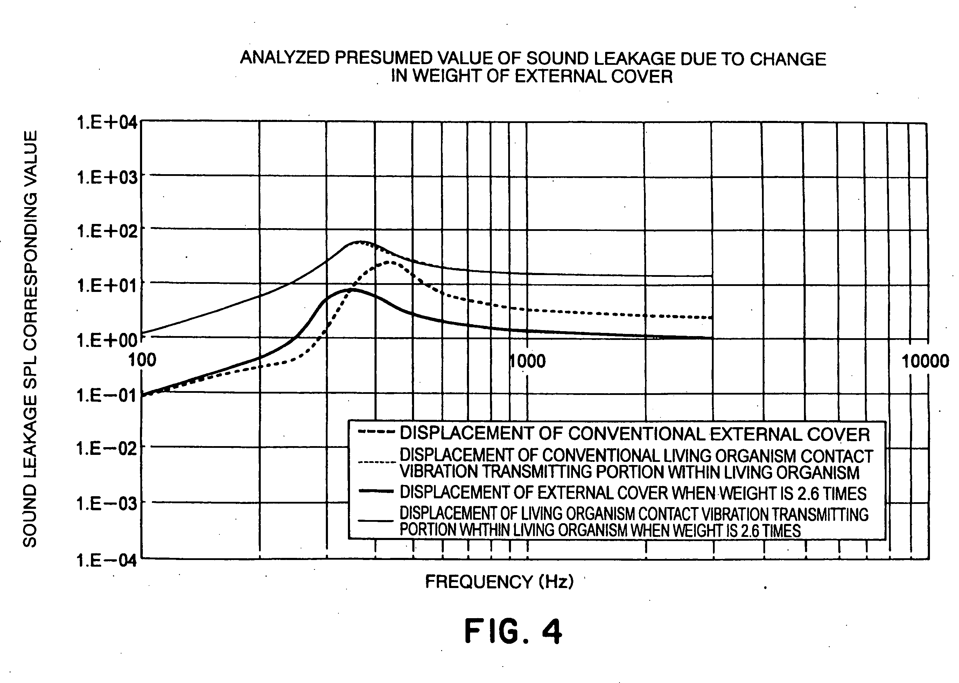

As shown in Fig. 4, it is presumed by analysis that no displacement of the living organism contact vibration transmitting portion 21 at this time is changed in comparison with the displacement prior to a change in weight.

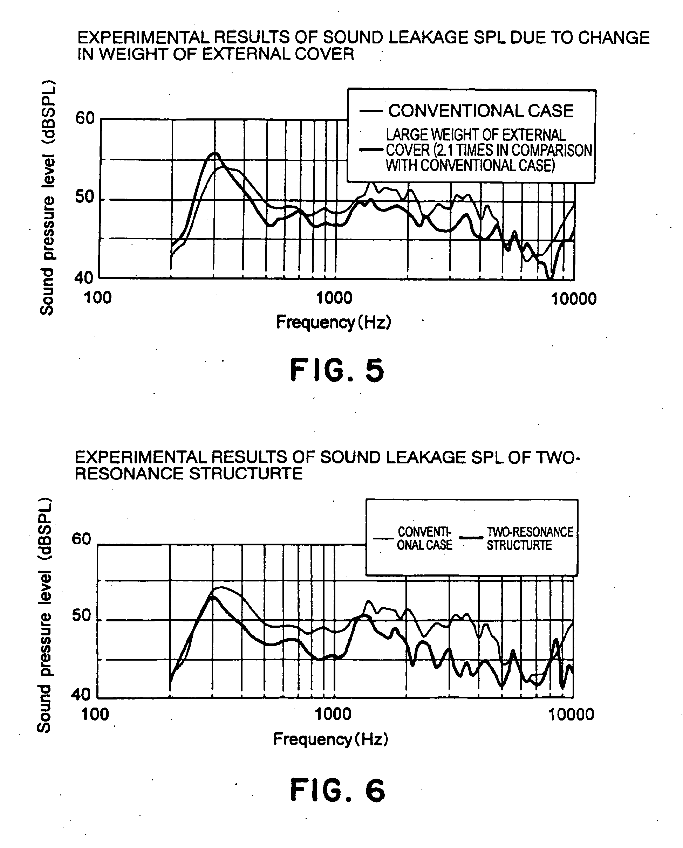

As shown in Fig. 5, it should be understood that noise reducing effects are also clearly large in experimental results.

In the invention, a corner portion of the external cover 15 is chamfered and an effective area of vibration transmission is reduced as a reduction in structural sound leakage. Further, the sound leakage is reduced by setting a vibration system of the communication signal transmitting portion 59 to a two-resonance structure.

Concretely, with reference to Fig. 2D, a first vibration system is constructed by the spiral spring 37 and the magnetic circuit 35 as a mass. A second vibration system is constructed by the spring 63 of a resin shape, the coil 39 and the base 23 as a mass.

In the first vibration system, a resonance frequency lies in the vicinity of 200 Hz. In the second vibration system, the resonance frequency is 10 kHz or more for reasons of cutoff on the high frequency area side of a received talk voice.

As shown in Fig. 6, it should be understood that the sound leakage is reduced in a frequency area from 200 to 8000 Hz in the two-resonance structure of the invention.

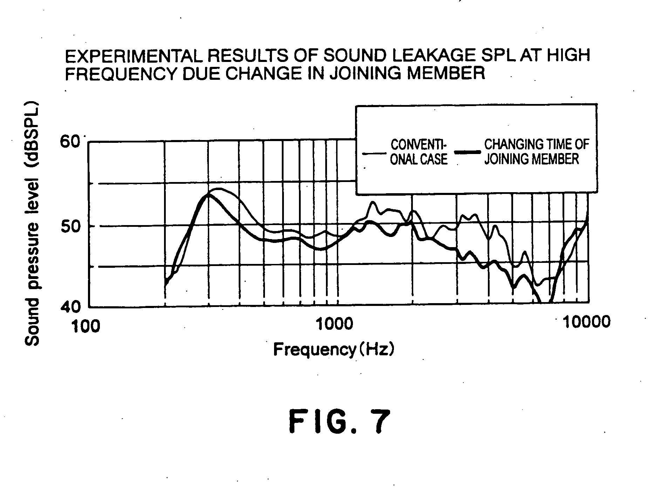

As shown in Fig. 7, a material 79 having a large Poisson's ratio (near 0.5), such as a double coated tape, is used in a joining portion of the first and second vibration systems, a joining portion of the second vibration system and the living organism contact vibration transmitting portion cover 25, or botch these joining portions. Accordingly, vibrational energy in a vibration transmitting direction is dispersed in a vertical direction, and the vibrational energy in a second mode (z-directional resonance of an arm of the spiral spring) of the first vibration system can be relaxed so that it contributes to the reduction in sound leakage at high frequency.

Further, in the invention, the vibration systems are made by constructional parts, such as an upper portion of the communication signal transmitting portion, the substrate 45 and a wiring 51 in the vicinity of the voice input portion. Further, a structure for dispersing vibration directivity is adopted by using the elastic adhesive 49 in the joining portion so as not to generate additional vibration noises. Further, vibration transmission is improved as experimental results by using a structure in which a spring of the first vibration system of the communication signal transmitting portion 59 is set to a spiral body and a spiral arm is extended in a direction perpendicular to a face of the spiral body. The vibration theoretically enters the interior (an area having a shape close to a linear shape) from an unstable portion (an area extending in a nonlinear shape in time) of the external surface of a living organism by giving an initial load to the living organism.

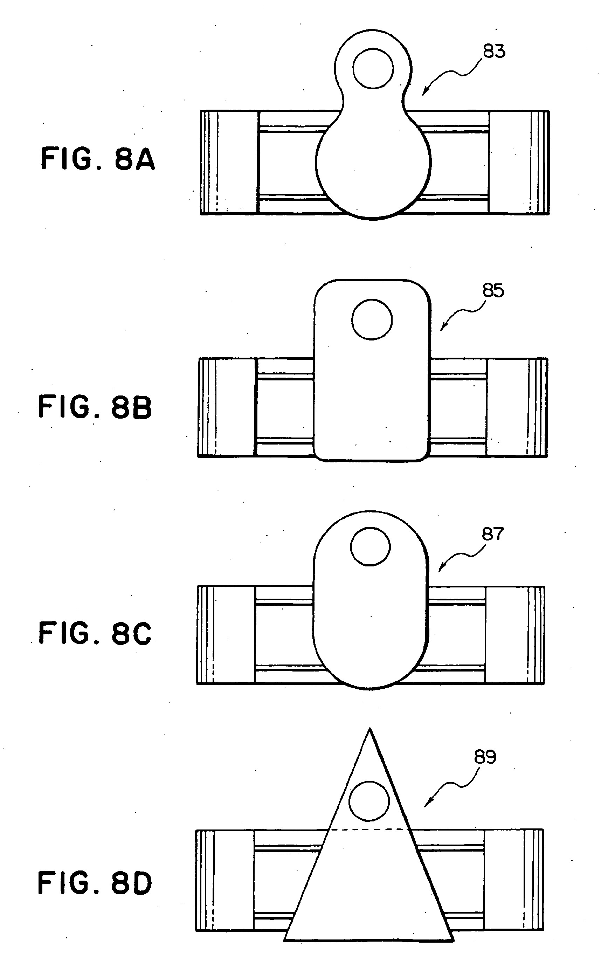

In the embodiment of the invention mentioned above, the external cover is approximately formed in an egg shape. However, as shown in Figs. 8A to 8D, effects similar to those in the embodiment of the invention are obtained even when the external cover is formed in a gourd shape 83, a square shape 85 having a round corner, an elliptical shape 87 and a triangular shape 89. Further, positions of the actuator and the wire may be located longitudinally and transversally. Further, similar to Fig. 2C, a circular hollow exposing the plate 27 of resin thereto, a metal case 75 corresponding to the microphone 43, and the gap 71 recessed by one stage from the cover are arranged on a bottom face side although these members are not shown in Figs. 8A to 8D.

As explained above, in accordance with the invention, it is possible to construct an optimal structure able to reduce sound leakage and improve transmission characteristics and control a vibration transmitting path of internal and external portions in the living organism conductive actuator.

## Claims

1. A living organism conductive actuator comprising:
an external portion (15)
a communication signal transmitting portion (59) for transmitting a communication signal by vibration via an operator's wrist, hand, hand rear portion, finger, or nail tip of a user when the communication signal transmitting portion is in contact with the operator's wrist, hand, hand rear portion, finger, or nail tip,
a voice input portion (61) provided laterally adjacent to the communication signal transmitting portion, and
two wires (13) having a spring property and mounted on an external portion (15) to support the living organism conductive actuator.

2. A living organism conductive actuator according to claim 1, further comprising a vibration-proof material (55) for vibration insulation on a living organism contact portion of the living organism conductive actuator except for said communication signal transmitting portion (69).

3. A living organism conductive actuator according to claim 1, wherein a viscoelastomer (65) is arranged in supporting portions of said two wires (13) having the spring property such that no additional resonance is generated.

4. A living organism conductive actuator according to any one of claims 1 to 3, wherein a living organism transmitting face of said communication signal transmitting portion (59) is constructed as a plate (27) of resin to reduce friction in a shearing direction.

5. A living organism conductive actuator according to one of claims 1 to 4, wherein a noninterference gap (71) is formed in a structure of said voice input portion (61) to avoid a mechanical interference except for a transmitted living organism.

6. A living organism conductive actuator according to one of claims 1 to 5, wherein said voice input portion (61) is arranged on a side face of said communication signal transmitting portion (59) from its upper portion so as to reduce a voice input noise.

7. A living organism conductive actuator according to one of claims 1 to 6, wherein a vibration proof material (41) for vibration insulation is added to the exterior of said voice input portion (61) to reduce a voice input noise from said communication signal transmitting portion (69).

8. A living organism conductive actuator according to one of claims 1 to 7, further comprising an external cover (15) manufactured by a metal for storing said communication signal transmitting portion (59), wherein a noise due to a mechanical load of said communication signal transmitting portion is restrained by this external cover manufactured by the metal.

9. A living organism conductive actuator according to one of claims 1 to 8, wherein the weight of a main body of the living organism conductive actuator (57) is set to several ten grams to reduce noise propagation due to a medium on an external surface.

10. A living organism conductive actuator according to one of claims 1 to 9, wherein a structure for chamfering a corner portion of an external cover (15) to reduce an effective area of vibration transmission is formed to reduce noise propagation due to a medium on a surface.

11. A living organism conductive actuator according to one of claims 1 to 10, wherein said communication signal transmitting portion (59) has a vibration system set to a two-resonance structure to reduce noise propagation due to a medium on an external surface.

12. A living organism conductive actuator.according to claim 11, wherein a material (79) having a large Poisson ratio near 0.5 is formed in a joining portion of the two-resonance system structure in said communication signal transmitting portion so that vibrational energy in a vibration transmitting direction is dispersed in a vertical direction, and the vibrational energy can be relaxed in a second mode of a first vibration system constructed by a spiral spring (35) and a magnetic circuit (35) constructed by a yoke (29), a magnet (31) and a plate (27) as a mass.

13. A living organism conductive actuator according to one of claims 1 to 12, wherein a spring of a first vibration system of said communication signal transmitting portion (59) is set to a spiral body and a spiral arm is extended in a direction perpendicular to a face of the spiral body.

14. A living organism conductive actuator according to one of claims 1 to 13, further comprising an external cover (15) made of a metal covering said voice input portion (61) and said communication signal transmitting portion (59).

## Patentansprüche

1. Leitender Aktuator für lebenden Organismus mit:
einem externen Abschnitt (15),
einem Kommunikationssignalübertragungsabschnitt (59) zum Übertragen eines Kommunikationssignals durch Vibration über eines Bedieners Handgelenk, Hand, Handrücken, Finger oder Nagelspitze eines Benutzers, wenn der Kommunikationssignalübertragungsabschnitt in Kontakt mit des Bedieners Handgelenk, Hand, Handrücken, Finger oder Nagelspitze ist,
einem Spracheingabeabschnitt (61), der seitlich angrenzend an den Kommunikationssignalübertragungsabschnitt bereitgestellt ist, und
zwei Drähten (13) mit einer Federeigenschaft, die an dem externen Abschnitt (15) angebracht sind, um den leitenden Aktuator für lebenden Organismus zu halten.

2. Leitender Aktuator für lebenden Organismus gemäß Anspruch 1, der weiter ein vibrationsfestes Material (55) zur Vibrationsisolation auf einem Kontaktabschnitt für lebenden Organismus des leitenden Aktuators für lebenden Organismus aufweist außer an dem Kommunikationssignalübertragungsabschnitt (69).

3. Leitender Aktuator für lebenden Organismus gemäß Anspruch 1, bei dem Viscoelastomer (65) an Halteabschnitten der zwei Drähte (13) mit der Federeigenschaft angeordnet ist, sodass keine zusätzliche Resonanz erzeugt wird.

4. Leitender Aktuator für lebenden Organismus gemäß einem der Ansprüche 1 bis 3, bei dem eine Übertragungsfläche für lebenden Organismus des Kommunikationssignalübertragungsabschnitt (59) als Platte (27) aus Harz aufgebaut ist, um die Reibung in einer Scherrichtung zu verringern.

5. Leitender Aktuator für lebenden Organismus gemäß einem der Ansprüche 1 bis 4, bei dem ein Nichtinterferenzspalt (71) in einem Aufbau des Spracheingabeabschnitts (61) gebildet ist, um eine mechanische Interferenz außer für einen übertragenen lebenden Organismus zu vermeiden.

6. Leitender Aktuator für lebenden Organismus gemäß einem der Ansprüche 1 bis 5, bei dem der Spracheingabeabschnitt (61) an einer Seitenfläche des Kommunikationssignalübertragungsabschnitts (59) von seinem oberen Abschnitt aus angeordnet ist, um eine Spracheingabestörung zu verringern.

7. Leitender Aktuator für lebenden Organismus gemäß einem der Ansprüche 1 bis 6, bei dem ein vibrationsfestes Material (41) zur Vibrationsisolation an dem Äußeren des Spracheingabeabschnitts (61) hinzugefügt ist, um eine Spracheingabestörung von dem Kommunikationssignalübertragungsabschnitts (69) zu verringern.

8. Leitender Aktuator für lebenden Organismus gemäß einem der Ansprüche 1 bis 7, der weiter eine externe Hülle (15) enthält, die aus einem Metall gefertigt ist, um den Kommunikationssignalübertragungsabschnitt (59) unterzubringen, wobei eine Störung aufgrund einer mechanischen Belastung des Kommunikationssignalübertragungsabschnitt durch diese externe Hülle, die aus einem Metall gefertigt ist, unterdrückt wird.

9. Leitender Aktuator für lebenden Organismus gemäß einem der Ansprüche 1 bis 8, bei dem das Gewicht eines Hauptkörpers des leitenden Aktuators für lebenden Organismus (57) auf einige zehn Gramm eingestellt ist, um eine Geräuschausbreitung aufgrund eines Mediums auf einer externen Oberfläche zu verringern.

10. Leitender Aktuator für lebenden Organismus gemäß einem der Ansprüche 1 bis 9, bei dem ein Aufbau des Abfasens eines Eckabschnitts einer externen Hülle (15) zum Verringern einer effektiven Fläche der Vibrationsübertragung gebildet ist, um eine Geräuschausbreitung aufgrund eines Mediums auf einer Oberfläche zu verringern.

11. Leitender Aktuator für lebenden Organismus gemäß einem der Ansprüche 1 bis 10, bei dem der Kommunikationssignalübertragungsabschnitt (59) ein Vibrationssystem aufweist, das auf einen Zweifachresonanzaufbau eingestellt ist, um eine Geräuschausbreitung aufgrund eines Mediums auf einer externen Oberfläche zu verringern.

12. Leitender Aktuator für lebenden Organismus gemäß Anspruch 11, bei dem ein Material (79) mit einer hohen Poissonzahl von etwa 0.5 in einem Verbindungsabschnitt des Zweifachresonanzsystemaufbaus des Kommunikationssignalübertragungsabschnitts gebildet ist, so dass eine Vibrationsenergie in einer Vibrationsübertragungsrichtung in einer Vertikalrichtung zerstreut ist und die Vibrationsenergie in einem zweiten Modus eines ersten Vibrationssystems relaxiert werden kann, das aus einer Spiralfeder (35) und einem Magnetkreis (35) gebildet ist, der aus einem Joch (29), einem Magneten (31) und einer Platte (27) als Masse aufgebaut ist.

13. Leitender Aktuator für lebenden Organismus gemäß einem der Ansprüche 1 bis 12, bei dem eine Feder eines ersten Vibrationssystems des Kommunikationssignalübertragungsabschnitts (59) auf einen Spiralkörper aufgesetzt ist und ein Spiralarm sich in einer Richtung senkrecht zu einer Fläche des Spiralkörpers erstreckt.

14. Leitender Aktuator für lebenden Organismus gemäß einem der Ansprüche 1 bis 13, der weiter eine externe Hülle (15) enthält, die aus einem Metall gefertigt ist und den Spracheingabeabschnitt (61) und den Kommunikationssignalübertragungsabschnitt (59) bedeckt.

## Revendications

1. Actionneur conducteur d'un organisme vivant comprenant:
une partie externe (15)
une partie (59) de transmission de signal de communication pour transmettre un signal de communication par vibration par l'intermédiaire d'un poignet, d'une main, du dos d'une main, d'un doigt d'un opérateur, ou de la pointe d'un ongle d'un utilisateur lorsque la partie de transmission de signal de communication est en contact avec le poignet, la main, le dos d'une main, le doigt, ou la pointe d'un ongle d'un opérateur,
une partie d'entrée vocale (61) pourvue de manière latéralement adjacente à la partie de transmission de signal de communication, et
deux fils (13) ayant une propriété de ressort et montés sur une partie externe (15) pour soutenir l'actionneur conducteur d'un organisme vivant.

2. Actionneur conducteur d'un organisme vivant selon la revendication 1, comprenant en plus un matériau résistant aux vibrations (55) pour l'isolation des vibrations sur une partie de contact d'un organisme vivant de l'actionneur conducteur d'un organisme vivant à l'exception de ladite partie (59) de transmission de signal de communication.

3. Actionneur conducteur d'un organisme vivant selon la revendication 1, dans lequel un élément viscoélastomère (65) est agencé dans des parties de soutien desdits deux fils (13) ayant la propriété de ressort de telle sorte qu'aucune résonance supplémentaire ne soit générée.

4. Actionneur conducteur d'un organisme vivant selon l'une quelconque des revendications 1 à 3, dans lequel une face de transmission d'un organisme vivant de ladite partie (59) de transmission de signal de communication est construite sous forme de plaque (27) d'une résine afin de réduire le frottement dans une direction de cisaillement.

5. Actionneur conducteur d'un organisme vivant selon l'une des revendications 1 à 4, dans lequel un jeu de non-interférence (71) est formé dans une structure de ladite partie d'entrée vocale (61) pour éviter une interférence mécanique sauf pour un organisme vivant transmis.

6. Actionneur conducteur d'un organisme vivant selon l'une des revendications 1 à 5, dans lequel ladite partie d'entrée vocale (61) est agencée sur une face latérale de ladite partie (59) de transmission de signal de communication à partir de sa partie supérieure de manière à réduire un bruit d'entrée vocale.

7. Actionneur conducteur d'un organisme vivant selon l'une des revendications 1 à 6, dans lequel un matériau résistant aux vibrations (41) pour l'isolation des vibrations est ajouté à la partie extérieure de ladite partie d'entrée vocale (61) pour réduire un bruit d'entrée vocale à partir de ladite partie (59) de transmission de signal de communication.

8. Actionneur conducteur d'un organisme vivant selon l'une des revendications 1 à 7, comprenant en plus un cache externe (15) réalisé en métal pour stocker ladite partie (59) de transmission de signal de communication, où un bruit induit par une charge mécanique de ladite partie de transmission de signal de communication est limité par ce cache externe réalisé en métal.

9. Actionneur conducteur d'un organisme vivant selon l'une des revendications 1 à 8, dans lequel le poids d'un corps principal de l'actionneur conducteur (57) d'un organisme vivant est réglé à plusieurs dizaines de grammes pour réduire une propagation de bruit provoqué par un support sur une surface externe.

10. Actionneur conducteur d'un organisme vivant selon l'une des revendications 1 à 9, dans lequel une structure pour chanfreiner une partie de coin du cache externe (15) pour réduire une section effective de transmission de vibrations est formée pour réduire la propagation de bruit induit par un vecteur sur une surface.

11. Actionneur conducteur d'un organisme vivant selon l'une des revendications 1 à 10, dans lequel ladite partie (59) de transmission de signal de communication a un système de vibrations établi à une structure à double résonance pour réduire la propagation de bruit induit par un vecteur sur une surface externe.

12. Actionneur conducteur d'un organisme vivant selon la revendication 11, dans lequel un matériau (79) qui possède un nombre de Poisson élevé proche de 0,5 est formé dans une partie de liaison de la structure du système à double résonnance dans ladite partie de transmission de signal de communication de sorte que l'énergie de vibration dans une direction de transmission de vibrations soit dispersée dans une direction verticale, et l'énergie de vibrations peut être relaxée dans un deuxième mode d'un premier système de vibrations construit par un ressort en hélice (35) et un circuit magnétique (35) construit par une culasse (29), un aimant (31) et une plaque (27) en tant que masse.

13. Actionneur conducteur d'un organisme vivant selon l'une des revendications 1 à 12, dans lequel un ressort d'un premier système de vibrations de ladite partie (59) de transmission de signal de communication est établi à un corps en spirale et un bras en spirale est déployé dans une direction perpendiculaire à une face du corps en spirale.

14. Actionneur conducteur d'un organisme vivant selon l'une des revendications 1 à 13, comprenant en plus un cache externe (15) réalisé en métal recouvrant ladite partie d'entrée vocale (61) et ladite partie (59) de transmission de signal de communication.
